# FASCICULE DE BREVET EUROPEEN

(11) **EP 3 416 709 B1**
(45) Date de publication et mention de la délivrance du brevet: **08.04.2020**
(21) Numéro de dépôt: 17708864.8
(22) Date de dépôt: 13.02.2017
(51) Int. Cl.: A61M 5/20, A61M 5/30, A61M 5/315, A61M 5/28

(54) **DISPOSITIF D'INJECTION SANS AIGUILLE À MEMBRANE CINTRÉE**
NADELLOSE INJEKTORVORRICHTUNG MIT GEKRÜMMTER MEMBRAN
NEEDLE-LESS INJECTOR DEVICE HAVING A CURVED MEMBRANE

(30) Priorité: 18.02.2016 FR 1651338
(43) Date de publication de la demande: 26.12.2018
(73) Titulaire: Crossject, 21000 Dijon (FR)
(72) Inventeur: VIGOT, Xavier, 21260 Veronnes (FR); AURIEL, Christophe, 21270 Binges (FR)
(74) Mandataire: Delorme, Nicolas
(86) Numéro de dépôt international: PCT/FR2017/050323
(87) Numéro de publication internationale: WO 2017/140974

(56) Documents cités:
- EP-A2- 2 366 418
- FR-A1- 2 852 517
- US-A- 3 802 430
- US-A1- 2003 114 789

## Description

L'invention concerne un dispositif d'injection sans aiguille comprenant une membrane dont la forme cintrée est adaptée pour favoriser son extension axiale dans le réservoir.

Le domaine technique de l'invention est celui des dispositifs d'injection sans aiguille, pré-remplis et jetables, fonctionnant avec une source d'énergie comme par exemple un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Un dispositif d'injection comporte de manière connue, comme par exemple dans la demande de brevet FR-A-2815544 (équivalente à WO 02/34317), un corps comprenant successivement un générateur de gaz, une chambre d'expansion, un réservoir contenant le principe actif liquide et un système d'injection.

Le réservoir est constitué par un tube en verre qui est inséré dans un logement tubulaire délimité par le corps du dispositif, le tube étant obturé par un piston supérieur, ou amont, et un piston inférieur, ou aval, entre lesquels est contenu le principe actif liquide.

L'extrémité libre inférieure du réservoir coopère avec une buse d'injection qui délimite au moins un canal d'injection s'étendant axialement suivant un axe d'injection.

La buse d'injection est délimitée axialement par une face supérieure en appui axial sur le réservoir, et une face inférieure d'injection adaptée pour coopérer avec un opercule de fermeture.

De plus, le dispositif d'injection comporte un capot creux qui enveloppe le corps et qui délimite une ouverture inférieure adaptée pour le passage de la buse d'injection.

Pour permettre l'injection du principe actif, le corps est monté coulissant dans le capot, de bas en haut suivant un axe de coulissement, entre une position de repos et une position d'injection, l'entraînement du corps étant réalisé lorsque l'utilisateur appui la buse d'injection sur sa peau.

Le déplacement du corps dans le capot permet le déclenchement du générateur de gaz, générant un gaz sous pression qui entraîne en déplacement les pistons pour injecter le principe actif à travers la peau du patient, en passant par la buse d'injection.

On connait un dispositif d'injection qui est équipé d'une membrane déformable élastiquement de forme globalement en T, qui comprend un disque annulaire radial qui est interposé axialement entre l'extrémité supérieure du réservoir et un siège formé par le corps, et une partie tubulaire qui s'étend axialement dans le réservoir, depuis le disque annulaire.

La partie tubulaire de la membrane est conçue pour s'étendre axialement sous l'effet du gaz sous pression, afin d'entraîner les pistons en déplacement.

La pression du gaz déforme également la membrane radialement, de sorte que le membrane vient en contact sur la paroi interne du réservoir en verre.

Le frottement entre la membrane, qui est généralement réalisée en élastomère, et la paroi en verre du réservoir, est important et absorbe une partie importante de l'énergie nécessaire à l'allongement et l'extension de la partie tubulaire de la membrane dans le réservoir.

Pour pallier ce problème, il est connu de lubrifier la membrane pour limiter le frottement entre la membrane et le réservoir.

Bien qu'efficace, la lubrification est une étape contraignante au cours de la réalisation et de l'assemblage du dispositif d'injection.

La présente invention vise notamment à résoudre cet inconvénient et se rapporte pour ce faire à un dispositif d'injection sans aiguille comportant :
- un corps formant un logement,
- un générateur de gaz,
- un réservoir tubulaire qui contient un principe actif à injecter, le réservoir s'étendant axialement dans ledit logement depuis une extrémité supérieure, jusqu'à une extrémité inférieure,
- une membrane déformable élastiquement de forme globalement en T, la membrane comprenant une partie tubulaire qui s'étend axialement dans le réservoir et qui est conçue pour s'allonger axialement dans le réservoir, sous l'effet de la pression générée par le générateur de gaz, et
- une buse d'injection du principe actif qui est agencée à l'extrémité inférieure du réservoir, ledit dispositif étant caractérisé en ce que la partie tubulaire de la membrane présente une forme globalement en entonnoir, ladite partie tubulaire présentant au moins :
- un tronçon supérieur de forme globalement tronconique de section décroissante suivant le sens d'allongement de la membrane, et
- un tronçon inférieur de forme globalement tronconique de section croissante suivant le sens d'allongement de la membrane, le tronçon supérieur présentant une longueur axiale supérieure à la longueur axiale du tronçon inférieur.

Une telle forme asymétrique en entonnoir favorise le déploiement de la membrane et son extension axiale dans le réservoir en limitant les frottements entre la membrane et la face interne du réservoir.

En effet, sur le tronçon supérieur de la membrane, la force exercée par le gaz sous pression s'applique perpendiculairement à la surface de la membrane, de sorte que la résultante axiale de cette force pousse la membrane vers le bas, suivant le sens d'écoulement du gaz sous pression.

Selon une autre caractéristique, la membrane est obturée par un fond qui présente la forme d'un disque globalement cylindrique qui est délimité axialement par une face supérieure plane en contact avec le gaz sous pression et une face inférieure plane.

La face supérieure plane du fond de la membrane offre une surface d'appui sur laquelle le gaz sous pression exerce une force orientée axialement, afin de favoriser l'allongement de la membrane.

De plus, le fond de la membrane présente une forme sensiblement cintrée qui délimite un espace radial avec une paroi interne du réservoir.

La forme cintrée du fond de la membrane permet de réduire les frottements entre le fond et la paroi du réservoir.

Selon un exemple de réalisation, la membrane est réalisée en matériau à base d'élastomère, de sorte que la membrane est déformable élastiquement.

Selon une autre caractéristique, la membrane comprend un disque annulaire radial qui est relié sur la partie tubulaire de la membrane, le disque annulaire étant en appui axial sur une extrémité supérieure du réservoir.

Selon une autre caractéristique, le réservoir est obturé par un piston supérieur et un piston inférieur entre lesquels est contenu le principe actif liquide, lesdits pistons étant adaptés pour être poussés axialement par la membrane sous l'effet de la pression générée par le générateur de gaz.

Selon une autre caractéristique, le principe actif contenu dans le réservoir est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

D'autres caractéristiques et avantages de l'invention apparaîtront à la lecture de la description détaillée qui suit pour la compréhension de laquelle on se reportera aux dessins annexés dans lesquels :
- la figure 1 est une vue en section axiale qui illustre un dispositif d'injection comportant la membrane en position de repos, selon l'invention ;
- la figure 2 est une vue en section axiale qui illustre le dispositif d'injection de la figure 1 avec la membrane en position allongée ;
- la figure 3 est une vue de détail en section axiale illustrant la membrane de la figure 1 agencée dans le réservoir.

Dans la description et les revendications, pour clarifier la description et les revendications, on adoptera à titre non limitatif la terminologie longitudinal, vertical et transversal en référence au trièdre L, V, T indiqué aux figures.

De plus, dans la présente demande, les termes « supérieur », « inférieur », « horizontal », « vertical », et leurs dérivés font référence à la position ou à l'orientation d'un élément ou d'un composant, cette position ou cette orientation étant considérée en référence à l'orientation du dispositif sur les figures et au trièdre L, V, T, sans référence à la gravité terrestre.

De même, les termes « axial » et « radial » doivent s'entendre en référence à l'axe B d'injection du dispositif d'injection.

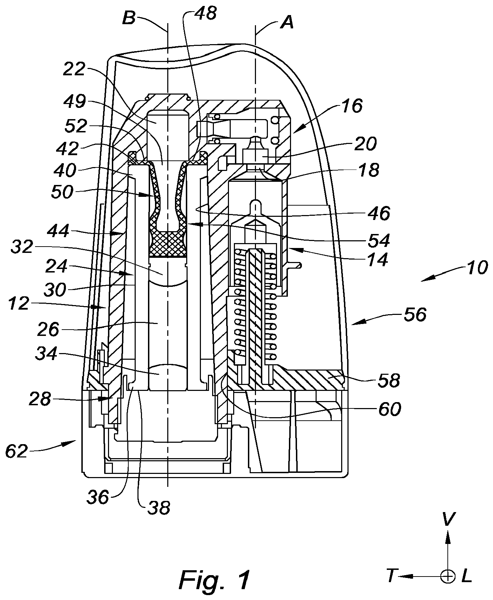

On a représenté à la figure 1 un dispositif 10 d'injection sans aiguille, ou seringue sans aiguille, qui comporte un corps 12 en forme de U comprenant successivement un dispositif de percussion 14, un générateur de gaz 16 comprenant une amorce 18 et une charge pyrotechnique 20, une chambre d'expansion 22, un réservoir 24 contenant le principe actif 26 liquide et une buse 28 d'injection.

Le dispositif de percussion 14 et le générateur de gaz 16 constituent un premier sous-ensemble linéaire du corps 12 qui s'étend axialement suivant un axe A de coulissement vertical, et le réservoir 24 contenant le principe actif 26 et la buse 28 d'injection forment un deuxième sous-ensemble linéaire du corps 12 qui s'étend axialement suivant un second axe B d'injection vertical.

Ces deux sous-ensembles sont reliés l'un à l'autre par la chambre d'expansion 22 qui a un axe perpendiculaire aux axes A, B des sous-ensembles.

Le réservoir 24 est constitué par un tube 30 en verre obturé par un piston supérieur 32 et un piston inférieur 34 entre lesquels est contenu le principe actif 26 liquide, les pistons étant réalisés en matériau déformable élastiquement à base d'élastomère.

Le réservoir 24 s'étend axialement depuis une collerette inférieure 36 qui présente une face inférieure 38 annulaire agencée en regard de la buse 28 d'injection, jusqu'à une collerette supérieure 40 présentant une face supérieure 42 annulaire.

Le réservoir 24 est agencé dans un logement 44 formé par le corps 12, logement 44 qui est délimité radialement par une paroi 46 tubulaire qui s'étend autour de l'axe B d'injection.

Le logement 44 s'étend axialement depuis un siège 48 radial supérieur qui est formé par le corps 12 et qui délimite un orifice de sortie 49 de la chambre d'expansion 22.

Selon un exemple de réalisation préféré, le corps 12 est réalisé par moulage en injection de matière plastique.

Aussi, selon la figure 1, le dispositif 10 est équipé d'une membrane 50 déformable élastiquement de forme globalement en T, qui comprend un disque 52 annulaire radial qui est interposé axialement entre la collerette supérieure 40 du réservoir 24 et le siège 48 formé par le corps 12, et une partie tubulaire 54 qui s'étend axialement dans le réservoir 24, depuis le disque 52 annulaire.

La partie tubulaire 54 de la membrane 50 est conçue pour s'étendre axialement, sous l'effet de la pression du gaz généré par le générateur de gaz 16, pour pousser le piston supérieur 32 vers le bas afin d'éjecter le principe actif 26 à travers la buse 28 d'injection.

A cet effet, la membrane 50 est réalisée dans un matériau à base d'élastomère. Plus particulièrement, la membrane 50 est réalisée en EPDM, c'est-à-dire en éthylène-propylène-diène monomère.

En référence à la figure 1, le corps 12 est enveloppé par un capot 56 creux qui délimite une ouverture inférieure fermée par une semelle 58 horizontale formant fond de capot.

La semelle 58 délimite un passage circulaire 60 autour de l'axe B d'injection qui est adapté pour le passage de la buse 28 d'injection et de l'extrémité inférieure du corps 12, de sorte que la buse 28 comporte un tronçon inférieur faisant saillie verticalement vers le bas hors du capot 56.

Plus particulièrement, la buse 28 est vissée sur une extrémité libre débouchante du logement 44 formé par le corps 12, la buse 28 comprimant axialement l'ensemble formé par le réservoir 24 et la membrane 50 sur le siège 48 du logement 44.

Aussi, le dispositif 10 d'injection est équipé d'un bouchon 62 qui est monté sur le corps 12 de façon amovible par un moyen de verrouillage du type à baïonnette.

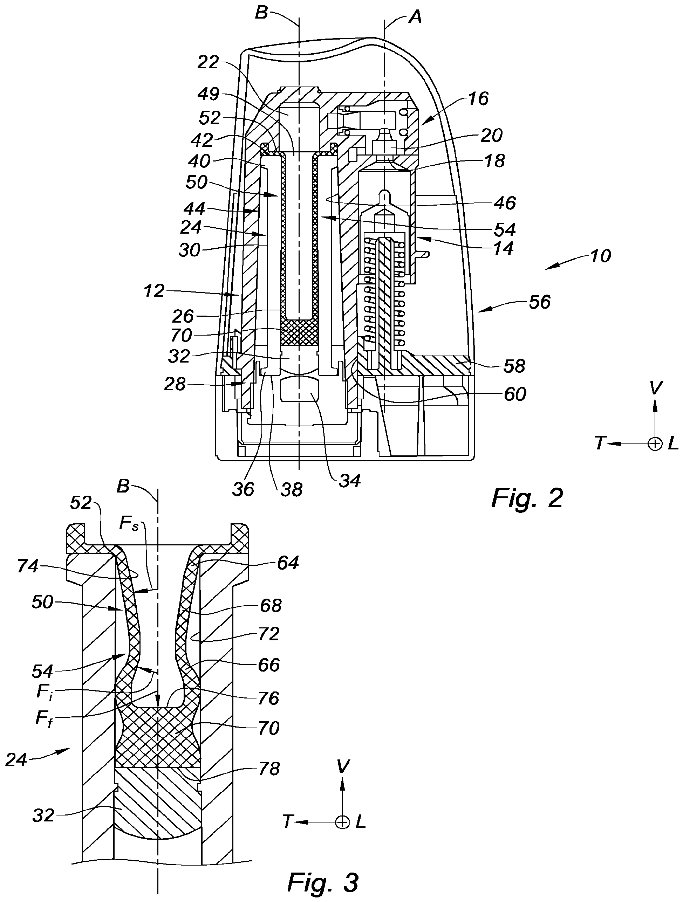

Conformément à l'invention, la partie tubulaire 54 de la membrane 50 présente une forme globalement en entonnoir, autrement dit une forme cintrée, comme on peut le voir à la figure 3.

La partie tubulaire 54 présente un tronçon supérieur 64, un tronçon inférieur 66, une portion de raccord 68 qui relie le tronçon supérieur 64 et le tronçon inférieur 66, et un fond 70.

On entend par « tronçon supérieur 64 » le tronçon amont suivant le sens d'écoulement des gaz dans le dispositif 10 d'injection, et par « tronçon inférieur 66 » le tronçon aval.

Le tronçon supérieur 64 présente une forme globalement tronconique de section décroissante suivant le sens d'allongement de la membrane 50, c'est-à-dire une section décroissante du haut vers le bas.

A l'inverse, le tronçon inférieur 66 présente une forme globalement tronconique de section croissante suivant le sens d'allongement de la membrane 50.

La portion de raccord 68 forme un rétrécissement radial de la membrane 50, de sorte que la surface de contact entre la partie tubulaire 54 de la membrane 50 et la paroi 72 interne du réservoir 24 est limitée, ce qui réduit les frottements entre la membrane 50 et le réservoir 24.

Le tronçon supérieur 64 de la partie tubulaire 54 présente une longueur axiale supérieure à la longueur axiale du tronçon inférieur 66.

Cette caractéristique permet de favoriser l'allongement axial de la membrane 50 sous l'effet de la pression générée par le générateur de gaz 16.

En effet, la force exercée par le gaz sous pression sur la membrane 50 s'applique perpendiculairement à la paroi interne 74 de la membrane 50.

Comme on peut le voir à la figure 3, la force exercée sur le tronçon supérieur 64 de la membrane 50 est illustrée par la flèche Fs, et la force exercée sur le tronçon inférieur 66 de la membrane 50 est illustrée par la flèche Fi.

La force Fs présente une résultante axiale orientée vers le bas, qui étire la membrane 50 vers le bas, et la force Fi présente une résultante axiale orientée vers le haut, qui s'oppose à la résultante axiale de la force Fs.

Etant donné que le tronçon supérieur 64 de la partie tubulaire 54 présente une longueur axiale supérieure à la longueur axiale du tronçon inférieur 66, le bilan des forces exercées sur la membrane 50 par le gaz sous pression tend à étendre la partie tubulaire 54 de la membrane 50 vers le bas.

De plus, la force Ff qui est exercé sur le fond 70 de la membrane 50 tend également à étendre la partie tubulaire 54 de la membrane 50 vers le bas.

Aussi, on constate que suivant le sens découlement du gaz sous pression du haut vers le bas, ou d'amont en aval, la pression s'exerce successivement sur le tronçon supérieur 64, puis ensuite sur le tronçon inférieur 66 de la membrane 50, ce qui favorise également l'allongement axial de la membrane 50.

Selon un autre aspect de l'invention, le fond 70 de la membrane 50 est relié sur le tronçon inférieur 66 de la partie tubulaire 54 de la membrane 50.

Le fond 70 présente la forme d'un disque globalement cylindrique qui est délimité axialement par une face supérieure 76 plane en contact avec le gaz sous pression et une face inférieure plane 78 en appui axial sur le piston supérieur 32.

Aussi, le fond 70 de la membrane 50 présente une forme sensiblement cintrée qui délimite un espace radial avec la paroi interne 72 du réservoir 24, pour limiter les frottements entre la membrane 50 et le réservoir 24.

## Revendications

1. Dispositif (10) d'injection sans aiguille comportant :
- un corps (12) formant un logement (44),
- un générateur de gaz (16),
- un réservoir (24) tubulaire qui contient un principe actif (26) à injecter, le réservoir (24) s'étendant axialement dans ledit logement (44) depuis une extrémité supérieure, jusqu'à une extrémité inférieure (36),
- une membrane (50) déformable élastiquement de forme globalement en T, la membrane (50) comprenant une partie tubulaire (54) qui s'étend axialement dans le réservoir (24) et qui est conçue pour s'allonger axialement dans le réservoir (24), sous l'effet de la pression générée par le générateur de gaz (16), et
- une buse (38) d'injection du principe actif (26) qui est agencée à l'extrémité inférieure du réservoir (24),
ledit dispositif (10) étant **caractérisé en ce que** la partie tubulaire (54) de la membrane (50) présente une forme globalement en entonnoir, ladite partie tubulaire (54) présentant au moins :
- un tronçon supérieur (64) de forme globalement tronconique de section décroissante suivant le sens d'allongement axial de la membrane (50), et
- un tronçon inférieur (66) de forme globalement tronconique de section croissante suivant le sens d'allongement axial de la membrane (50), le tronçon supérieur (64) présentant une longueur axiale supérieure à la longueur axiale du tronçon inférieur (66).

2. Dispositif (10) d'injection sans aiguille selon la revendication 1, **caractérisé en ce que** la membrane (50) est obturée par un fond (70) qui présente la forme d'un disque globalement cylindrique qui est délimité axialement par une face supérieure (76) plane en contact avec le gaz sous pression et une face inférieure plane (78).

3. Dispositif (10) d'injection sans aiguille selon la revendication 2, **caractérisé en ce que** le fond (70) de la membrane (70) présente une forme sensiblement cintrée qui délimite un espace radial avec une paroi interne (72) du réservoir (24).

4. Dispositif (10) d'injection sans aiguille selon l'une quelconque des revendications précédentes, **caractérisé en ce que** la membrane (50) est réalisée en matériau à base d'élastomère.

5. Dispositif (10) d'injection sans aiguille selon l'une quelconque des revendications précédentes, **caractérisé en ce que** la membrane (50) comprend un disque (52) annulaire radial qui est relié sur la partie tubulaire (54) de la membrane (50), le disque (52) annulaire étant en appui axial sur une extrémité supérieure du réservoir (24).

6. Dispositif (10) d'injection sans aiguille selon l'une quelconque des revendications précédentes, **caractérisé en ce que** le réservoir est obturé par un piston supérieur (32) et un piston inférieur (34) entre lesquels est contenu le principe actif (26) liquide, lesdits pistons (32, 34) étant adaptés pour être poussés axialement par la membrane (50) sous l'effet de la pression générée par le générateur de gaz (16).

7. Dispositif (10) d'injection sans aiguille selon l'une quelconque des revendications précédentes, **caractérisé en ce que** le principe actif (26) contenu dans le réservoir (24) est choisi parmi le groupe comprenant les principes actifs suivants :
- Methotrexate,
- Adrénaline,
- Sumatriptan,
- Hydrocortisone,
- Naloxone,
- Midazolam,
- Apomorphine,
- Ethylnatrexone bromide,
- Phytomenadione,
- Chlorpromazine hydrocloride,
- Zuclopenthixol acetate,
- Danaparoid sodium,
- Enoxaparin sodium,
- Estradiol cypionate,
- Medoxyprogesterone acetate,
- Medroparin calcium,
- Methylprednisolone acetate
- Heparin calcium,
- Terbuline.

## Patentansprüche

1. Nadellose Injektionsvorrichtung (10), Folgendes beinhaltend:
- einen Körper (12), der eine Aufnahme (44) bildet,
- einen Gasgenerator (16),
- einen rohrförmigen Tank (24), der einen zu injizierenden Wirkstoff (26) enthält, wobei sich der Tank (24) axial in der Aufnahme (44) von einem oberen Ende bis zu einem unteren Ende (36) erstreckt,
- eine elastisch verformbare Membran (50), in einer im Allgemeinen T-Form, wobei die Membran (50) einen rohrförmigen Teil (54) umfasst, der sich axial in dem Tank (24) erstreckt, und der gestaltet ist, um sich axial in dem Tank (24) unter der Wirkung des Drucks, der durch den Gasgenerator (16) generiert wird, auszudehnen, und
- eine Injektionsdüse (38) des Wirkstoffes (26), die am unteren Ende des Tanks (24) angeordnet ist,
wobei die Vorrichtung (10) **dadurch gekennzeichnet ist, dass** der rohrförmige Teil (54) der Membran (50) eine im Allgemeinen trichterförmige Form aufweist, wobei der rohrförmige Teil (54) mindestens Folgendes aufweist:
- ein oberes Teilstück (64) in einer im Allgemeinen kegelstumpfförmigen Form mit abnehmendem Querschnitt in der axialen Ausdehnungsrichtung der Membran (50), und
- ein unteres Teilstück (66) in einer im Allgemeinen kegelstumpfförmigen Form mit zunehmendem Querschnitt in der axialen Ausdehnungsrichtung der Membran (50), wobei das obere Teilstück (64) eine axiale Länge größer als die axiale Länge des unteren Teilstücks (66) aufweist.

2. Nadellose Injektionsvorrichtung (10) nach Anspruch 1, **dadurch gekennzeichnet, dass** die Membran (50) durch einen Boden (70) verschlossen ist, der die Form einer im Allgemeinen zylindrischen Scheibe aufweist, die axial durch eine ebene Oberseite (76) in Kontakt mit dem unter Druck stehenden Gas, und eine ebene Unterseite (78) eingegrenzt ist.

3. Nadellose Injektionsvorrichtung (10) nach Anspruch 2, **dadurch gekennzeichnet, dass** der Boden (70) der Membran (70) eine im Wesentlichen gebogene Form aufweist, die einen radialen Raum mit einer Innenwand (72) des Tanks (24) eingrenzt.

4. Nadellose Injektionsvorrichtung (10) nach einem der vorstehenden Ansprüche, **dadurch gekennzeichnet, dass** die Membran (50) aus einem Material auf Elastomer-Basis gefertigt ist.

5. Nadellose Injektionsvorrichtung (10) nach einem der vorstehenden Ansprüche, **dadurch gekennzeichnet, dass** die Membran (50) eine radiale ringförmige Scheibe (52) umfasst, die auf dem rohrförmigen Teil (54) der Membran (50) angeschlossen ist, wobei die ringförmige Scheibe (52) in axialer Anlage an einem oberen Ende des Tanks (24) ist.

6. Nadellose Injektionsvorrichtung (10) nach einem der vorstehenden Ansprüche, **dadurch gekennzeichnet, dass** der Tank von einem oberen Kolben (32) und einem unteren Kolben (34) verschlossen ist, zwischen denen der flüssige Wirkstoff (26) enthalten ist, wobei die Kolben (32, 34) angepasst sind, um axial durch die Membran (50) unter der Wirkung des durch den Gasgenerator (16) generierten Drucks angeschoben zu werden.

7. Nadellose Injektionsvorrichtung (10) nach einem der vorstehenden Ansprüche, **dadurch gekennzeichnet, dass** der in dem Tank (24) enthaltene Wirkstoff (26) aus der Gruppe ausgewählt wird, die die folgenden Wirkstoffe umfasst:
- Methotrexat,
- Adrenalin,
- Sumatriptan,
- Hydrocortison,
- Naloxon,
- Midazolam,
- Apomorphin,
- Ethylnatrexonbromid,
- Phytomenadion,
- Chlorpromazinhydrochlorid,
- Zuclopenthixol-Acetat,
- Danaparoid-Natrium,
- Enoxaparin-Natrium,
- Extradiol-Cypionat,
- Medoxyprogesteronacetat,
- Medroparin-Kalzium,
- Methylprednisolonacetat,
- Heparin-Calcium,
- Terbulin.

## Claims

1. A needleless injection device (10) including:
- a body (12) forming a housing (44),
- a gas generator (16),
- a tubular reservoir (24) which contains an active ingredient (26) to be injected, the reservoir (24) extending axially into said housing (44) from an upper end, to a lower end (36),
- a generally T-shaped elastically deformable diaphragm (50), the diaphragm (50) comprising a tubular portion (54) which extends axially into the reservoir (24) and which is designed so as to extend axially into the reservoir (24), under the effect of the pressure generated by the gas generator (16), and
- a nozzle (38) for injecting the active ingredient (26) which is arranged at the lower end of the reservoir (24),
said device (10) being **characterized in that** the tubular portion (54) of the diaphragm (50) has a generally funnel-like shape, said tubular portion (54) having at least:
- one generally frustoconical shaped upper segment (64) with a decreasing section in the axial direction of elongation of the diaphragm (50), and
- one generally frustoconical shaped lower segment (66) with an increasing section in the axial direction of elongation of the diaphragm (50), the upper segment (64) having an axial length larger than the axial length of the lower segment (66).

2. The needleless injection device (10) according to claim 1, **characterized in that** the diaphragm (50) is sealed by a bottom (70) which has the shape of a generally cylindrical disc which is axially delimited by a planar upper face (76) in contact with the pressurized gas and a planar lower face (78).

3. The needleless injection device (10) according to claim 2, **characterized in that** the bottom (70) of the diaphragm (70) has a substantially curved shape which delimits a radial space with an inner wall (72) of the reservoir (24).

4. The needleless injection device (10) according to any one of the preceding claims, **characterized in that** the diaphragm (50) is made of an elastomer-based material.

5. The needleless injection device (10) according to any one of the preceding claims, **characterized in that** the diaphragm (50) comprises a radial annular disc (52) which is connected to the tubular portion (54) of the diaphragm (50), the annular disc (52) bearing axially on an upper end of the reservoir (24).

6. The needleless injection device (10) according to any one of the preceding claims, **characterized in that** the reservoir is sealed by an upper plunger (32) and a lower plunger (34) between which the liquid active ingredient (26) is contained, said plungers (32, 34) being adapted to be pushed axially by the diaphragm (50) under the effect of the pressure generated by the gas generator (16).

7. The needleless injection device (10) according to any one of the preceding claims, **characterized in that** the active ingredient (26) contained in the reservoir (24) is selected from the group comprising the following active ingredients:
- Methotrexate,
- Adrenaline,
- Sumatriptan,
- Hydrocortisone,
- Naloxone,
- Midazolam,
- Apomorphine,
- Ethylnatrexone bromide,
- Phytomenadione,
- Chlorpromazine hydrochloride,
- Zuclopenthixol acetate,
- Danaparoid sodium,
- Enoxaparin sodium,
- Estradiol cypionate,
- Medoxyprogesterone acetate,
- Medroparin calcium,
- Medroxyprogesterone acetate
- Heparin calcium,
- Terbulin.
